# EUROPÄISCHE PATENTSCHRIFT

(11) **EP 1 362 092 B1**
(45) Veröffentlichungstag und Bekanntmachung des Hinweises auf die Patenterteilung: **26.09.2007**
(21) Anmeldenummer: 02708249.4
(22) Anmeldetag: 11.02.2002
(51) Int. Cl.: C12N 5/00, A61L 27/24, A61L 27/38

(54) **VERFAHREN ZUR HERSTELLUNG EINES BIOLOGISCHEN GEWEBES UNTER VERWENDUNG EINER KOLLAGENUNTERLAGE UND ZUGEHÖRIGES GEWEBEKONSTRUKT**
METHOD FOR THE PRODUCTION OF A BIOLOGICAL TISSUE USING A COLLAGEN SUPPORT AND CORRESPONDING TISSUE CONSTRUCT
PROCEDE DE PRODUCTION D'UN TISSU BIOLOGIQUE A L'AIDE D'UN SUPPORT EN COLLAGENE ET PRODUIT DE SYNTHESE TISSULAIRE

(30) Priorität: 13.02.2001 DE 10106512
(43) Veröffentlichungstag der Anmeldung: 19.11.2003
(73) Patentinhaber: corLife GbR, 30625 Hannover (DE)
(72) Erfinder: HAVERICH, Axel, 30916 Isernhagen (DE); KOFIDIS, Theo, 30559 Hannover (DE)
(74) Vertreter: Läufer, Martina
(86) Internationale Anmeldenummer: PCT/DE2002/000527
(87) Internationale Veröffentlichungsnummer: WO 2002/064753

(56) Entgegenhaltungen:
- WO-A-00/23008
- WO-A-00/67672
- WO-A-98/54301
- WO-A-99/47188
- DE-A- 19 828 726

## Beschreibung

Die Erfindung betrifft ein Verfahren zur Herstellung eines biologischen Gewebes, bei welchem Muskel Zellen auf einer Unterlage kultiviert werden, die Verwendung einer bestimmten Unterlage zu diesem Zweck sowie ein für die Implantation geeignetes Gewebekonstrukt , das mit dem Verfahren erhältlich ist.

Zu verschiedenen Zwecken wird heute versucht, Gewebe, Organe oder Teile davon nachzuzüchten, um durch Krankheit oder Unfall geschädigte oder sogar zerstörte Gewebe und Organe ersetzen zu können. Derartige Versuche gibt es u.a. für Haut, Lebergewebe und Herzmuskelgewebe.

Zum Stützen des Gewebes - insbesondere in der Anfangsphase der Kultur-und zum anfänglichem Verteilen der Zellen werden oft flüssige Kollagene, z.B. ein unter dem Handelsnamen "Matrigel^{®}" erhältliches Kollagen, oder auch biologisch abbaubare Feststoffe verwendet. Die bisher erzielten Ergebnisse sind jedoch bezüglich der Gewebequalität noch unzureichend. Probleme bereitet besonders die Konsolidierung der Matrix, ungleichmäßige Zellverteilung in dieser Matrix und damit verbunden eine ungleichmäßige nicht naturähnliche Gewebeausbildung.

Aus der DE 198 28 726 A1 ist ein bioartifizielles Transplantat bekannt, das auf Basis einer nicht-denaturierten, praktisch zellfreien Collagen-Matrix mit aufgelockerter Struktur erhalten wird. Für diese Matrix wird vorab ein allogenes oder xenogenes Gewebe enzymatisch oder chemisch von fremden Zellen befreit.

Die WO 99/47188 offenbart die Verwendung einer Biopolymermatte für eine Gewebereparatur oder Geweberekonstruktion. Die Matte kann Zellen oder Makromoleküle für Zellwachstum, Morphogenese, Differenzierung oder Gewebebildung enthalten.

Bei der Nachzüchtung von Herzgeweben kommt hinzu, dass eine ungleichmäßige, den natürlichen Verhältnissen nicht genügend entsprechende Verteilung der Herzzellen zu einem ungleichmäßigen und asynchronen Kontraktionsverhalten führt.

Für die Bereitstellung nachgezüchteter Herzgewebe besteht ein großes Bedürfnis. Einem Großteil der Gesamtmorbidität und Mortalität der Bevölkerung liegen kardiovaskuläre Ursachen zugrunde. Die medizinische Forschung sucht daher nach neuen Verfahren zum Gewebs- und Organersatz, um die Folgen des Gewebeuntergangs und des Organfunktionsverlustes (z.B. Herzinfarkt) im kardiovaskulären Bereich einzudämmen. Die Produktion von Herzmuskelgewebe im Bioreaktor ist daher das Ziel intensiver Forschungsaktivitäten.

Die Aufgabe der Erfindung besteht darin, ein möglichst naturähnlich ausgebildetes dreidimensionales Gewebekonstrukt bereitzustellen, das für den Ersatz oder teilweisen Ersatz von Geweben oder Organen, speziell von Muskel- und

Herzmuskelgewebe geeignet ist.
Insbesondere soll ein solches Konstrukt geschaffen werden, das als Grundlage für einen Zell- oder Gewebetransfer in ein erkranktes oder zerstörtes Organ durch Implantation in den menschlichen oder tierischen Körper dienen kann.
Ferner ist ein Verfahren zur Herstellung eines solchen Konstruktes anzugeben.

Zur Lösung dieser Aufgabe ist bei einem Verfahren der eingangs genannten Art vorgesehen, dass als Unterlage ein industriell hergestelltes filzartiges Gewebe aus Kollagenfibrillen, das als Kollagenpflaster zur Blutstillung oder Wundversorgung im Handel erhältlich ist, vorgelegt wird, dass Herzmuskelzellen auf die Unterlage aufgebracht oder in sie eingebracht werden, dass die Kultivierung in einem Nährmedium erfolgt und dass die Herzmuskelzellen auf der Unterlage kultiviert werden bis die Kollagenfasern zum überwiegenden Teil abgebaut sind und sich ein synchron kontrahierendes Herzmuskelgewebestück gebildet hat.

Ein entscheidender Aspekt der Erfindung besteht darin, dass eine Unterlage für die Zell- oder Gewebekultur gefunden werden konnte, die, wie sich herausgestellt hat, für die gleichmäßige Ausbildung eines Gewebeverbundes ideal geeignet ist. Diese Unterlage besteht aus einem industriell hergestellten netz- oder filzartigen Gewebe aus Kollagenfasern oder auch "Kollagenfibrillen", wie es zur Blutstillung und Wundversorgung, insbesondere im operativen Bereich, seit vielen Jahren verwendet wird. Derartige als Unterlage für die Herstellung des erfindungsgemäßen Gewebekonstrukts nach dem erfindungsgemäßen Verfahren geeignete Kollagenpflaster sind im Handel erhältlich, beispielsweise sind die unter den Handelsnamen "Collatamb®" und "TissuFleece®" bekannten Kollagenpflaster geeignet.

Die Kollagenunterlage bildet eine filz- oder schwammartige Struktur, die mit Flüssigkeiten getränkt werden kann, bzw. sich mit diesen vollsaugt. Es ist daher leicht möglich das Kollagengewebe mit Zellen, die sich beispielsweise in Nährlösung befinden können, zu beimpfen.

Da sich die filz- oder schwammartige Unterlage in Kulturmedium ebenso wie im Körper langsam auflöst, kommt es während der Kultuvierung zu einer gleichmäßigen Verteilung und fortschreitenden Ausdehnung der Zellen in der Unterlage oder Matrix. Im Verlauf der Kultur bildet sich dadurch ein gleichmäßig solides, mechanisch stabiles Gewebe.

Die Kultur wird soweit fortgeführt bis die Kollagenfasern zum überwiegenden Teil abgebaut sind und sich ein gleichförmig ausgebildetes Herzmuskelgewebe gebildet hat. Es ergibt sich ein gleichmäßiges, solides, mechanisch stabiles und synchron kontrahierendes Herzmuskelgewebestück.

Ein solches Herzmuskelgewebestück ist für die Implantation geeignet. Es kann auch vorteilhaft für Forschungszwecke verwendet werden, da es leicht reproduzierbar und zuverlässig in seinen mechanischen und physikalischen Eigenschaften ist. Das auf diese Weise hergestellte Herzmuskelgewebe bietet sich daher auch für wissenschaftliche Untersuchungen an Herzgeweben an und ist für Forschungslaboratorien von großem Nutzen, beispielsweise auch in der Pharmaforschung.

Die Kultivierung der Zellen auf dem abbaubaren Kollagengerüst nach dem erfindungsgemäßen Verfahren erfolgt in einem Nährmedium, vorzugsweise in Dulbeccos modified eagle medium (DMEM). Dabei kann so vorgegangen werden, dass die Kollagenunterlage in eine Schale eingelegt wird und dass die Zellen in Nährmedium suspendiert aufgegeben werden. Die Zellen können auch in dem Nährmedium vorgelegt werden, woraufhin die Kollagenunterlage hierein eingelegt wird. Die Zellen können auch in die Unterlage eingespritzt werden, ggf. mehrfach und/oder an verschiedenen Stellen der Unterlage. Ebenso können die vorstehend angegebenen Verfahren zum Auf- und Einbringen der Zellen auf oder in die Unterlage in geeigneter Weise kombiniert werden.

In Weiterbildung der Erfindung wird das Verfahren so durchgeführt, dass die Kultivierung in dreidimensionaler Form in einer passenden Negativ-Form erfolgt. Im einfachsten Fall kann die Form ein Quader oder eine Scheibe sein. Beispielsweise könnte die Kollagengewebeunterlage in der Form passend für eine kleine Petrischale zugeschnitten und in diese eingelegt werden. Die Zellen können in dem Kulturmedium vorgelegt und/oder nach Einlegen der Unterlage aufgegeben werden.

Die Zellen können auch im Gemisch mit weiteren Zusätzen wie z.B. Antibiotika aufgegeben und/oder in die Unterlage eingebracht werden.

Vorzugsweise können den Zellen weiterhin Faktoren und/oder Co-Faktoren zugegeben werden, beispielsweise vascular endothelial growth factor (VEGF), fibroplast growth factor (FGF), thrombocyte growth factor-b (TGF-b).

Das erfindungsgemäße Gewebekonstrukt ist vorzugsweise so ausgebildet, dass die Zellen Teil eines auf und/oder in der Unterlage kultivierten zusammenhängenden Zellgewebes sind, d.h., die Kultur wurde in vitro so weit geführt, dass ein zusammenhängendes Zellgewebe erzeugt wurde.

Im folgenden wird die Erfindung anhand eines in der Zeichnung dargestellten Beispiels näher erläutert. In der Zeichnung zeigen:
- Fig. 1:: eine Schnittansicht durch eine Zellsuspension enthaltende Kulturschale for Einlegen der Kollagenunterlage;
- Fig. 2:: ein nach der Kultur gemäß Fig. 1 erhaltenes Gewebekonstrukt.

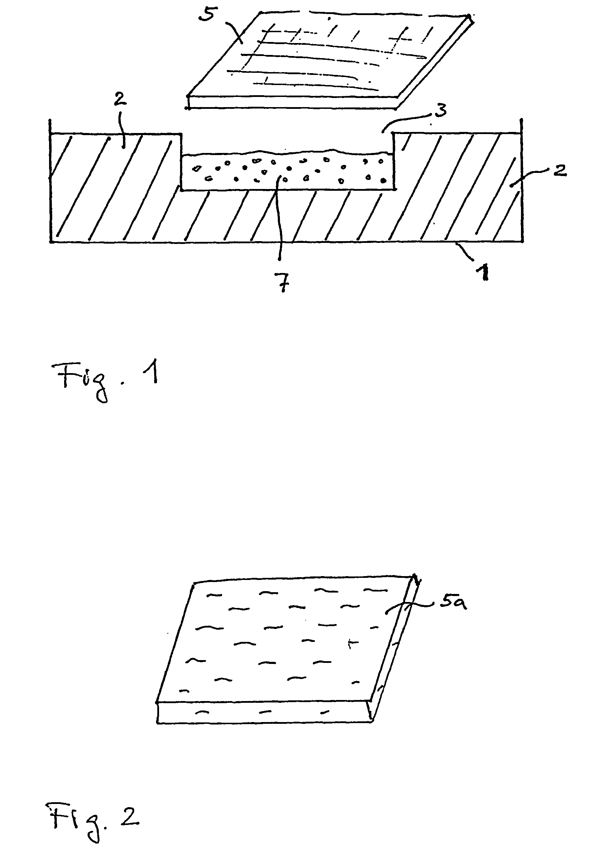

Fig 1 zeigt eine Petrischale 1, die mit Silikon 2 ausgegossen ist. Aus dem Silikon wurde als Negativ-Form 3 für das zu züchtende Gewebestück ein quaderförmiges Stück ausgeschnitten, welches gerade so bemessen ist, dass eine aus TissuFleece^{®} passend zugeschnittene Kollagen-Gewebeunterlage 5 eingelegt werden kann. Anstelle eines quaderförmigen Ausschnitts kann auch eine andere passende Form gewählt werden, die sich nach dem gewünschten Einsatzzweck des fertigen gezüchteten Gewebes richtet.

In dem Ausschnitt bzw der Negativ-Form 3 befindet sich in dieser Darstellung bereits ein Gemisch aus Herzzellen (Kardiomyocyten) und einem Medium, wie Dulbeccos modified eagle medium (DMEM), fetal calf serum (FCS), Penicillin/Streptomycin (P/S). Beim Einlegen der Unterlage 5 dringt das Gemisch 7 in die Unterlage ein und durchtränkt diese. Zusätzlich können, wenn gewünscht, nach dem Einlegen der Unterlage 5 weitere Zellen von oben aufgegeben werden.

Die weitere Kultur erfolgt unter üblichen und vom Fachmann nach bekannten Kriterien einstellbaren Bedingungen. Im vorliegenden Fall waren die Kulturbedingungen wie folgt:
Temperierung im Brutschrank bei 37 °C, Nährmedium: Dulbeccos modified eagle medium (DMEM)
Zusätze: fetal calf serum (FCS), Penicillin/Streptomycin (P/S), 5 % CO₂.

Kultur über 84 Tage ergab ein mechanisch stabiles Herzgewebestück, wie es in Fig. 2 als 5a dargestellt ist. Die ursprüngliche Unterlage 5 hat sich bereits weitgehend aufgelöst, und es ist ein zusammenhängendes, festes Herzgewebestück, was ohne Schwierigkeiten aus der Form entnommen werden und der weiteren Verwendung - Implantation oder wissenschaftliche Untersuchung - zugeführt werden kann, entstanden.

Das frisch entnommene gezüchtete Gewebe zeigt eine gleichmäßige Zellstruktur und ist selbstkontrahierend.
Es ist dehnbar und plastisch, ähnlich dem nativen Herzen. Das Gewebe ist mechanisch stabil genug für die Handhabung und verschiedenste Manipulationen. Es zeigt eine langsame Abbaukinetik (Tage). Ferner ist das Gewebe elektrisch stimulierbar und rhythmisch kontrahierend.

## Patentansprüche

1. Verfahren zur Herstellung eines biologischen Gewebes, bei welchem Muskelzellen auf einer Unterlage kultiviert werden,
**dadurch gekennzeichnet,**
**dass** als Unterlage ein industriell hergestelltes filzartiges Gewebe aus Kollagenfibrillen, das als Kollagenpflaster zur Blutstillung oder Wundversorgung im Handel erhältlich ist, vorgelegt wird, dass Herzmuskelzellen auf die Unterlage aufgebracht oder in sie eingebracht werden, dass die Kultivierung in einem Nährmedium erfolgt und dass die Herzmuskelzellen auf der Unterlage kultiviert werden bis die Kollagenfasern zum überwiegenden Teil abgebaut sind und sich ein synchron kontrahierendes Herzmuskelgewebestück gebildet hat.

2. Verfahren nach Anspruch 1, **dadurch gekennzeichnet, dass** die Kultivierung in Dulbeccos modified eagle medium (DMEM) erfolgt.

3. Verfahren nach Anspruch 1 oder 2, **dadurch gekennzeichnet, dass** die Zellen in Nährmedium suspendiert aufgegeben und/oder die Unterlage eingespritzt werden.

4. Verfahren nach einem der Ansprüche 1 bis 3, **dadurch gekennzeichnet, dass** die Kultivierung in dreidimensionaler Form in einer passenden Negativ-Form erfolgt.

5. Verfahren nach einem der Ansprüche 1 bis 4, **dadurch gekennzeichnet, dass** den Zellen Substanzen, vorzugsweise Faktoren und Co-Faktoren zugegeben werden.

6. Verfahren nach Anspruch 5, **dadurch gekennzeichnet, dass** die Faktoren gefäßwachstumsfördernde Faktoren sind.

7. Gewebekonstrukt aus Herzmuskelzellen, erhältlich durch ein Verfahren gemäß einem der Ansprüche 1 bis 6.

## Claims

1. Method for the production of a biological tissue in which muscle cells are cultured on a support, **characterized in that** an industrially produced felt-like fabric of collagen fibrils which is commercially available as collagen patch for haemostasis or wound management is provided as support, **in that** myocardial cells are applied to the support or introduced therein, **in that** the culturing takes place in a nutrient medium, and **in that** the myocardial cells are cultured on the support until the collagen fibres have been predominantly degraded and a synchronously contracting piece of myocardial tissue has formed.

2. Method according to Claim 1, **characterized in that** the culturing takes place in Dulbecco's modified eagle medium (DMEM).

3. Method according to Claim 1 or 2, **characterized in that** the cells are suspended in nutrient medium and/or injected into the support.

4. Method according to any of Claims 1 to 3, **characterized in that** the culturing takes place in 3-dimensional form in an appropriate negative mould.

5. Method according to any of Claims 1 to 4, **characterized in that** substances, preferably factors and cofactors, are added to the cells.

6. Method according to Claim 5, **characterized in that** the factors are vessel growth-promoting factors.

7. Tissue construct of myocardial cells obtainable by a method according to any of Claims 1 to 6.

## Revendications

1. - Procédé de production d'un tissu biologique, dans lequel des cellules musculaires sont cultivées sur un support, **caractérisé en ce qu'**on présente en tant que support un tissu feutré fabriqué industriellement en fibrilles de collagène disponible dans le commerce en tant qu'emplâtre de collagène pour étancher le rang ou pour soigner les plaies, **en ce qu'**on dépose sur le support, ou on introduit dans celui-ci des cellules de muscle cardiaque, **en ce qu'**on effectue la culture dans un milieu nutritif et **en ce que** les cellules de muscle cardiaque sont cultivées sur le support jusqu'à ce qu'une partie prépondérante des fibres de collagène soient décomposées et qu'une pièce de tissu du muscle cardiaque se contractant de manière synchrone se soit formée.

2. - Procédé selon la revendication 1, **caractérisé en ce que** la culture est effectuée dans le milieu eagle modifié de Dulbecco (DMEM).

3. - Procédé selon la revendication 1 ou 2, **caractérisé en ce que** les cellules sont déposées en suspension dans du milieu nutritif et/ou injectées dans le support.

4. - Procédé selon l'une des revendications 1 à 3, **caractérisé en ce que** la culture est effectuée sous forme tridimensionnelle dans une forme négative convenable.

5. - Procédé selon l'une des revendications 1 à 4, **caractérisé en ce que** des substances, de préférence des facteurs et des co-facteurs, sont ajoutées aux cellules.

6. - Procédé selon la revendication 5, **caractérisé en ce que** les facteurs sont des facteurs favorables à la croissance des vaisseaux.

7. - Structure tissulaire en cellules de muscle cardiaque pouvant être obtenue par un procédé selon l'une des revendications 1 à 6.
